# EUROPEAN PATENT SPECIFICATION

(11) **EP 2 349 207 B1**
(45) Date of publication and mention of the grant of the patent: **13.05.2015**
(21) Application number: 09818803.0
(22) Date of filing: 28.09.2009
(51) Int. Cl.: A61K 47/10, A61K 47/26, A61K 47/36, A61K 9/00, A61K 9/08, A61K 31/167

(54) **LIQUID PHARMACEUTICAL FORMULATION CONTAINING PARACETAMOL**
FLÜSSIGE PHARMAZEUTISCHE FORMULIERUNG MIT PARACETAMOL
FORMULATION PHARMACEUTIQUE LIQUIDE CONTENANT DU PARACÉTAMOL

(30) Priority: 09.10.2008 EP 08425654
(43) Date of publication of application: 03.08.2011
(73) Proprietor: Aziende Chimiche Riunite Angelini Francesco A.C.R.A.F. S.p.A., 00181 Roma (IT)
(72) Inventor: MARIOTTI, Francesca, I-61100 Pesaro (PU) (IT); SCARPETTI, Paolo, I-60015 Falconara Marittima (AN) (IT); RAGNI, Lorella, I-60033 Chiaravalle (AN) (IT); VALENTI, Mauro, I-20013 Magenta (MI) (IT)
(74) Representative: Allaix, Roberto
(86) International application number: PCT/EP2009/062501
(87) International publication number: WO 2010/040652

(56) References cited:
- WO-A-03/047502
- US-A1- 2006 093 629
- US-A1- 2007 166 336
- US-A1- 2008 085 892

## Description

### FIELD OF THE INVENTION

The present invention relates to a liquid pharmaceutical formulation containing paracetamol.

More particularly, the present invention relates to a sugar-free liquid pharmaceutical formulation containing an aqueous solution of paracetamol, a solubilizing agent containing polyethylene glycol, a thickening agent containing xanthan gum, and a sweetening system containing sucralose and a mixture of polyols.

### PRIOR ART

It is known in the prior art that from the physicochemical standpoint paracetamol is an odourless white powder, with a particularly bitter taste, moderately soluble in water (1.4 g/100 ml at 20°C) with a pH of the resultant solution moderately acid between 5.0 and 6.5.

From the chemical standpoint, paracetamol is a derivative of p-aminophenol that corresponds to the following formula:

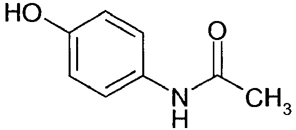

From the pharmacological standpoint, paracetamol is widely used as an analgesic with pronounced antipyretic activity. Owing to this particular activity it is used in various pharmaceutical forms with a wide dosage range.

Paracetamol has a mechanism of action that is linked to its ability to inhibit the synthesis of the prostaglandins, in particular inhibiting the activity of cyclooxygenase.

Administration by the oral route is the preferred method of administration of paracetamol, like any other active principle, whether liquid or solid, as it proves quicker and more practical for the end user.

The pharmaceutical forms used can be solid and liquid.

In general, the particularly bitter taste of many active principles does not constitute a serious problem for the administration of solid pharmaceutical forms. In these cases it is possible to mask the bitter taste of the active principles by coating the pharmaceutical form, whether the latter is in the form of a tablet or in the form of a capsule, with synthetic polymers or with a sugar coating.

However, the solid pharmaceutical form is particularly disadvantageous when the end user is a child or an elderly person with problems with swallowing or when the dosage of the active principle is linked in particular to the patient's weight, as in the case of antibiotics, anti-inflammatories and antipyretics, the dose of which is closely related to the patient's weight and age.

In these cases it is particularly convenient and practical to administer the active principle in a liquid pharmaceutical form, for example in the form of syrup or drops. The liquid preparations are represented by suspensions and solutions depending on whether the active principle is suspended or dissolved in the medium. In the case of active principles with a particularly unpleasant taste and/or of very low solubility, often pharmaceutical forms are used in which the active principle is suspended, rather than dissolved.

US patent 5,409,907 describes an example of a pharmaceutical form in which the active principle is not dissolved, but is in suspension. The suspension described in said patent contains, per 100 ml, from 0.5 to 1.0 g of microcrystalline cellulose, from 0.1 to 0.2 g of xanthan gum, up to 90 g of sweetening sugars, from 10 to 30 g of water, and up to 40 g of active principle.

However, the pharmaceutical forms in which the active principle is in suspension notoriously present various disadvantages connected above all with sedimentation of the disperse phase over time and with the instability of said dispersion. Sedimentation causes a change in the concentration of the active principle within the suspension. The instability of the suspension leads to separation and stratification of the components of the suspension. This leads to difficulty and uncertainty in the administration of the correct dose of active principle, especially bearing in mind that the end users, in particular in the case of elderly persons, do not take care or are not able to shake the bottle well before use to restore a homogeneous distribution of the active principle in the suspension.

It is therefore clear that it would be preferable to administer liquid pharmaceutical forms in which the active principle is completely dissolved, but to achieve this in the case of active principles with a particularly unpleasant taste and/or of very low solubility it is necessary to have formulations containing special solubilizing and sweetening agents.

US patent 5,154,926 describes a syrup containing, per 100 ml, from 0.5 to 5 g of paracetamol or phenobarbital, from 5 to 30 g of a polyol (glycerol, propylene glycol) or a polymer thereof (polyethylene glycols or polypropylene glycols with molecular weight between 300 and 400), from 0.5 to 5 g of a water-soluble macromolecule (polyvinyl pyrrolidones), from 10 to 60 g of sugar sweeteners, and water sufficient to reach 100 ml (q.s.).

Document US2006093629 discloses sugar-free paracetamol suspensions comprising sorbitol, glycerol, sucralose and xanthan gum.

Patent WO03/047502 describes a liquid formulation in which the active principle is dissolved or dispersed in an aqueous medium containing polyvinylpyrrolidone and/or copolyvidone (a copolymer of vinylpyrrolidone and vinyl acetate) and a polyethylene glycol of high molecular weight. The formulations containing acetaminophen (a synonym of paracetamol) are always described as suspensions, and include amounts of sugar sweeteners between 20 and 95 wt.%.

Solutions and suspensions containing polyvinylpyrrolidone are subject to severe darkening over time, to a greater extent and more rapidly with higher storage temperature, caused by the formation of colouring substances resulting from the oxidation of the pyrrolidone group.

Moreover, the high content of sugar sweeteners makes it inappropriate to administer these solutions and suspensions to subjects who do not wish to ingest high-energy substances or who are, for a variety of reasons, restricted to particular low-sugar diets, for example diabetic subjects.

### SUMMARY OF THE INVENTION

The applicant found, surprisingly, that a sugar-free liquid pharmaceutical formulation containing an aqueous solution of paracetamol, a solubilizing agent containing polyethylene glycol, a thickening agent containing xanthan gum, and a sweetening system containing sucralose and a mixture of polyols including glycerol, sorbitol and xylitol makes it possible to overcome the aforementioned problems.

In particular, the applicant found that the pharmaceutical formulation of the present invention makes it possible to maintain in solution an amount of paracetamol up to 5% w/v relative to the total volume of said pharmaceutical formulation.

The applicant found that the pharmaceutical formulation of the present invention is stable over time without exhibiting the phenomena of darkening that are typical of formulations containing polyvinylpyrrolidone and without exhibiting phenomena of precipitation of the active principle.

Moreover, the applicant found, surprisingly, that although containing paracetamol in solution and being completely free of sugar sweeteners, the pharmaceutical formulation of the present invention still has an excellent and particularly agreeable palatability, comparable to the palatability of conventional syrups containing paracetamol.

Therefore, in a first aspect the present invention relates to a sugar-free liquid pharmaceutical formulation containing an aqueous solution of paracetamol, a solubilizing agent containing polyethylene glycol, a thickening agent containing xanthan gum, and a sweetening system containing sucralose and a mixture of polyols including glycerol, sorbitol and xylitol in an amount between approx. 15% and 35% w/v relative to the total volume of said pharmaceutical formulation.

In a second aspect, the present invention relates to a pharmaceutically acceptable sugar-free liquid excipient for the administration of active principles of unpleasant taste containing an aqueous vehicle, a solubilizing agent containing polyethylene glycol, a thickening agent containing xanthan gum, and a sweetening system containing sucralose and a mixture of polyols including glycerol, sorbitol and xylitol in an amount between approx. 15% and 35% w/v relative to the total volume of said pharmaceutical formulation.

The expression "sugar-free" is intended to define an excipient/formulation without a determinable amount of sugars, while the term "sugars" is intended to include all the natural monosaccharides and disaccharides, commonly defined as saccharides or reducing sugars, such as glucose, fructose, and sucrose (although the latter does not have reducing capacity). The expression "% w/v relative to the total volume" is intended to define the number of parts by weight relative to 100 parts by volume, typically expressed in grams per 100 millilitres, but equivalent to kilograms per 100 litres.

### DETAILED DESCRIPTION OF THE INVENTION

The present invention, in at least one of the aforementioned aspects, can show one or more of the preferred characteristics described below.

The sugar-free liquid pharmaceutical formulation of the present invention comprises an aqueous solution of paracetamol, a solubilizing agent containing polyethylene glycol, a thickening agent containing xanthan gum, and a sweetening system containing sucralose and a mixture of polyols including glycerol, sorbitol and xylitol in an amount between approx. 15% and 35% w/v relative to the total volume of said pharmaceutical formulation.

Preferably, the liquid pharmaceutical formulation of the present invention includes an amount of paracetamol up to 5% w/v, more preferably up to 4% w/v, and even more preferably up to 3% w/v relative to the total volume of said pharmaceutical formulation. Typically, the optimum amount of paracetamol included in the liquid pharmaceutical formulation of the present invention is between 2% and 3% w/v relative to the total volume of said pharmaceutical formulation.

The applicant observed that below the aforementioned maximum limit, the amount of paracetamol in the liquid pharmaceutical formulation of the present invention remains stably in solution, without giving the pharmaceutical formulation an unpleasant taste, and permitting a convenient optimum dose of active principle.

Advantageously, the solubilizing agent used in the liquid pharmaceutical formulation of the present invention is a polyethylene glycol of high molecular weight, preferably greater than 1,000, more preferably between 2,000 and 10,000, and even more preferably between 3,000 and 8,000. Typically, the polyethylene glycol used in the liquid pharmaceutical formulation of the present invention is PEG 4000 or PEG 6000, the latter proving to be the polyethylene glycol with the highest solubilizing capacity. The polyethylene glycols that can be used in the present invention are commercial products, distributed for example by Alfa Aesar GmbH, Karlsruhe, Germany, and CarboMer, Inc., San Diego, California, USA.

Preferably, the liquid pharmaceutical formulation of the present invention includes an amount of polyethylene glycol greater than 10% w/v, and more preferably greater than 15% w/v relative to the total volume of said pharmaceutical formulation. Typically, the optimum amount of polyethylene glycol included in the liquid pharmaceutical formulation of the present invention is between 15% and 20% w/v relative to the total volume of said pharmaceutical formulation.

Conveniently, the thickening agent used in the liquid pharmaceutical formulation of the present invention is xanthan gum, a commercial polysaccharide extracted by a process of fermentation of glucose and/or sucrose carried out in the presence of Xanthomonas campestris, and marketed for the first time around the 1960s by the CP Kelco Company, USA. Currently, xanthan gum is marketed by the CP Kelco Company, USA with the trade names KELTROL^{®}, KELZAN^{®} and XANTURAL^{®}. Preferably, the xanthan gum used in the liquid pharmaceutical formulation of the present invention is the product XANTURAL^{®} 75.

Preferably the liquid pharmaceutical formulation of the present invention contains an amount of xanthan gum between 0.1 % and 2.0% w/v, more preferably between 0.2% and 1.0% w/v relative to the total volume of said pharmaceutical formulation.

The sweetening system used in the liquid pharmaceutical formulation of the present invention comprises sucralose and a mixture of polyols containing glycerol, sorbitol, and xylitol.

Advantageously, the liquid pharmaceutical formulation of the present invention contains an amount of sucralose between 0.05% and 0.5% w/v, more preferably between 0.075% and 0.25% w/v relative to the total volume of said pharmaceutical formulation.

Preferably, the liquid pharmaceutical formulation of the present invention contains an amount of mixture of polyols between 20% and 30% w/v, more preferably between 22% and 28% w/v relative to the total volume of said pharmaceutical formulation.

According to a preferred embodiment of the present invention the sweetening system comprises a mixture of glycerol, xylitol, and sorbitol in a total amount between 23% and 27% w/v relative to the total volume of said pharmaceutical formulation.

Advantageously, the pharmaceutical formulation of the present invention comprises a sweetening system represented by 7% to 14% w/v of glycerol, from 7% to 14% w/v of sorbitol, and from 3% to 7% w/v of xylitol relative to the total volume of said pharmaceutical formulation.

The applicant observed that the combination of xanthan gum, sucralose and polyols of the present invention, surprisingly, made it possible to mask the unpleasant taste of paracetamol, giving the liquid pharmaceutical formulation of the present invention an agreeable taste and consistency, comparable to that of a sugar-based syrup.

In particular, the applicant observed that the combination of xanthan gum, sucralose and polyols of the present invention, surprisingly, isolated contact of paracetamol with the taste buds, imparting a pleasant taste particularly suitable for the paediatric age group and a consistency comparable to that of a true sucrose-based syrup.

The liquid pharmaceutical formulation of the present invention can moreover contain other pharmaceutically acceptable additives conventionally known by a person skilled in the art, for example preservatives, antioxidants, buffering agents, stabilizers, colorants and flavourings.

Useful examples of preservatives and antioxidants are sorbic acid, sodium sorbate and potassium sorbate, methyl-p-hydroxybenzoate (methylparaben), ethyl-p-hydroxybenzoate (ethylparaben), and propyl-p-hydroxybenzoate (propylparaben), ascorbic acid, sodium ascorbate or potassium ascorbate, gallic acid and sodium or potassium gallates, or mixtures thereof. The mixture of potassium sorbate and methylparaben is particularly preferred.

Useful examples of buffering agents are organic and inorganic acid-base buffer systems, for example, citric acid and citrates of sodium or potassium, phosphoric acid and phosphates of sodium and of potassium. The combination citric acid and sodium citrate is particularly preferred.

Useful examples of stabilizers include alginic acid and alginates of sodium and potassium, agar-agar, carrageenin, and gum tragacanth.

Useful examples of flavouring agents include natural or synthetic flavours, for example strawberry flavour, mandarin flavour, peach flavour, lemon flavour, raspberry flavour, and mixtures thereof.

The amounts of each of the aforementioned additives are generally between 0.01% and 2.0% w/v, more preferably between 0.05% and 1.0% w/v relative to the total volume of said pharmaceutical formulation.

According to a particularly preferred embodiment the liquid pharmaceutical formulation of the present invention contains the ingredients of the following Table A in amounts between the minimum value and the maximum value shown in said Table A. The amounts are all expressed in grams (g), apart from the amount of demineralized water, which is expressed in millilitres (ml).

**TABLE A**

| Ingredient | minimum | maximum |
|---|---|---|
| Paracetamol | 2.0 | 4.0 |
| PEG 6000 | 15.0 | 20.0 |
| Xanthan gum | 0.3 | 0.5 |
| Glycerol | 8.0 | 12.0 |
| Sorbitol | 8.0 | 12.0 |
| Xylitol | 4.0 | 6.0 |
| Sucralose | 0.08 | 0.12 |
| Buffering system | 0.50 | 1.00 |
| Preserving system | 0.25 | 0.35 |
| Flavours | 0.25 | 0.50 |
| Demineralized water | q.s. 100 | |

In a further aspect, the present invention relates to a pharmaceutically acceptable sugar-free liquid excipient for the administration of active principles of unpleasant taste comprising an aqueous vehicle, a solubilizing agent containing polyethylene glycol, a thickening agent containing xanthan gum, and a sweetening system containing sucralose and a mixture of polyols that includes glycerol, sorbitol and xylitol in an amount between approx. 15% and 35% w/v relative to the total volume of said pharmaceutical formulation.

The active principles of unpleasant taste that can be administered advantageously in dissolved form in the pharmaceutically acceptable liquid excipient of the present invention are active principles containing hydrophilic groups capable of forming hydrogen bonds, for example the hydroxyl group, the carboxyl group, and the amino group. In particular, said active principles can be, as non-limiting examples, ibuprofen, phenylpropanolamine hydrochloride, pseudoephedrine hydrochloride, phenylephrine hydrochloride, diphenhydramine hydrochloride, guaifenesin, dextromethorphan hydrobromide, clorpheniramine maleate, brompheniramine maleate, terfenadine, loratadine, bromexine hydrochloride, ambroxol hydrochloride, salbutamol sulphate, amoxicillin, ampicillin, cloxacillin, flucloxacillin, cephalexin, and combinations thereof.

The pharmaceutically acceptable sugar-free liquid excipient of the present invention, in at least one of its aspects, can display one or more of the preferred characteristics described previously for the pharmaceutical formulation of the present invention.

### EXAMPLE 1

Formulations 1 and 2 were prepared by mixing the amounts of the components stated in the following Table 1 according to the following procedure.

The PEG6000 and the methyl p-hydroxybenzoate were dissolved in demineralized water heated to 80°C. The temperature of the resultant solution was lowered to 60°C, then the potassium sorbate was added and dissolved. The temperature of the resultant solution was lowered to 40°C, then the paracetamol was added and dissolved. The temperature of the resultant solution was lowered to 25°C, then the citric acid, the sodium citrate, and the sweetening system (saccharin sodium and sucrose for formulation 1, sucralose, xylitol, sorbitol and glycerol for formulation 2) were added and dissolved. The flavours and the xanthan gum were then added to the clear solution and dissolved, and finally the solution was made up to a volume of 100 ml with demineralized water at 25°C.

The amounts of the components in Table 1 are all expressed in grams (g), apart from the amount of demineralized water, which is expressed in millilitres (ml).

**TABLE 1**

| Composition | 1 | 2 |
|---|---|---|
| Paracetamol | 2.4 | 2.4 |
| PEG 6000 | 16.0 | 16.0 |
| Citric acid | 0.25 | 0.25 |
| Sodium citrate | 0.51 | 0.51 |
| Potassium sorbate | 0.18 | 0.18 |
| Methyl p-hydroxybenzoate | 0.13 | 0.13 |
| Xantural^{®}75 | - | 0.4 |
| Glycerol | - | 10.0 |
| Sorbitol | - | 10.0 |
| Xylitol | - | 5.0 |
| Sucralose | - | 0.1 |
| Saccharin sodium | 0.15 | - |
| Sucrose | 35.0 | - |
| Strawberry flavour | 0.12 | 0.12 |
| Mandarin flavour | 0.24 | 0.24 |
| Demineralized water | q.s. 100 | q.s. 100 |

Xantural^{®}75 is the trade name of a xanthan gum made by CP Kelco Company, USA (http://www.cpkelco.com/).

### EXAMPLE 2

Formulations 1 and 2 prepared according to Example 1 were submitted to a palatability test in order to verify their acceptability by end users.

Thirty subjects were selected, aged between twenty and fifty years. The test was performed by instructing the selected subjects to perceive and assess, with a score from 0 to 3, the relative stimuli to bitterness, tingling, clogging and viscidity at the time of administration (T₀), during swallowing (T₁), immediately after swallowing (T₂), and five minutes after swallowing (T₃). The following total scores were calculated for each subject:
- total score of the individual stimuli resulting from the sum of the scores obtained at T₀, T₁, T₂ and T₃.
- total score obtained on adding together the total scores of the individual stimuli.

The following Table 2 summarizes the average results obtained, which were analysed with the Wilcoxon signed-rank statistical method.

**TABLE 2**

| Formulation | 1 | 2 | Value of p |
|---|---|---|---|
| Bitterness | 3.47 | 3.27 | n.d. |
| Tingling | 1.20 | 1.37 | n.d. |
| Clogging | 2.73 | 3.87 | =0.07 |
| Viscidity | 2.47 | 4.33 | <0.05 |

Statistical analysis showed:
- a statistically significant difference (p<0.05) between the two syrups with reference to the sensation of viscidity, owing to the greater oiliness of the product (mean 2=4.33 vs. mean 1-2.47); and
- a difference at the limit of significance (p=0.07) between the two syrups with reference to the sensation of clogging, owing to the greater consistency of the product (mean 2=3.87 vs. mean 1=2.73).

The values relating to the sensation of viscidity and consistency, despite the presence of a thickening agent such as xanthan gum for modulating product viscosity and consistency, were only slightly less pleasant in formulation 2, and were still considered acceptable.

For the other stimuli analysed (bitterness and tingling) and for the sum total of the stimuli, statistically significant differences were not found between the two syrups.

The selected subjects were interviewed and were asked to make an overall assessment of the product (unacceptable, acceptable, good, excellent), as well as of the need to drink water after administration (yes, no).

The following Tables 3 and 4 summarize the results obtained, which were analysed by the McNemar statistical method. All the values are expressed in numerical percentages.

**TABLE 3**

| Formulation 1 | | | | |
|---|---|---|---|---|
| Overall assessment | Unacceptable | Acceptable | Good | Excellent |
| | 10.0 | 46.7 | 33.3 | 10.0 |
| | | | | |
| Necessary to drink water | Yes | | No | |
| | 50 | | 50 | |

**TABLE 4**

| Formulation 2 | | | | |
|---|---|---|---|---|
| Overall assessment | Unacceptable | Acceptable | Good | Excellent |
| | 13.3 | 56.7 | 26.7 | 3.3 |
| | | | | |
| Necessary to drink water | Yes | | No | |
| | 56.7 | | 43.3 | |

Regarding the need to drink water and the overall assessment expressed by the subjects for the two syrups, no statistically significant differences were found.

### EXAMPLE 3

Formulation 2 of the present invention was submitted to an assessment of stability in various conditions of temperature and relative humidity.

The results are summarized in the following Table 5

**TABLE 5**

| **Time** | **Appearance** | **pH** | **Viscosity (cP)** | **Paracetamol (% of theoretical)** |
|---|---|---|---|---|
| Start | Viscous, opalescent, colourless solution | 5.18 | 76 | 100.4% |

| 25°C 60% RH | | | | |
|---|---|---|---|---|
| 1 month | Complies | 5.20 | 77 | 99.7 |
| 6 months | Complies | 5.20 | 77 | 101.1 |

| 30°C 65% RH | | | | |
|---|---|---|---|---|
| 1 month | Complies | 5.21 | 82 | 99.7 |
| 6 months | Complies, with very slight yellowing | 5.21 | 78 | 99.9 |

| 40°C 75% RH | | | | |
|---|---|---|---|---|
| 1 month | Complies, with slight yellowing | 5.21 | 82 | 99.7 |
| 6 months | Complies, with slight yellowing | 5.17 | 75 | 99.7 |

| 50°C 75% RH | | | | |
|---|---|---|---|---|
| 1 month | Complies, with slight yellowing | 5.20 | 82 | 99.7 |

The data in Table 5 demonstrated the stability of formulation 2 of the present invention even in the most critical storage conditions.

### EXAMPLE 4

For the purpose of determining the best sweetening system, the following formulations were prepared and tested, varying the number and the amounts of the components of the system.

Formulations 3 to 10 were prepared by mixing the amounts of the components shown in the following Table 6 by the same procedure as in Example 1. The amounts of the components in Table 6 are all expressed in grams (g), apart from the amount of demineralized water, which is expressed in millilitres (ml).

**TABLE 6**

| Composition | 3 | 4 | 5 | 6 | 7 | 8 | 9 | 10 |
|---|---|---|---|---|---|---|---|---|
| Paracetamol | 2.4 | 2.4 | 2.4 | 2.4 | 2.4 | 2.4 | 2.4 | 2.4 |
| PEG 6000 | 16.0 | 16.0 | 16.0 | 16.0 | 16.0 | 16.0 | 16.0 | 16.0 |
| Citric acid | 0.25 | 0.25 | 0.25 | 0.25 | 0.25 | 0.25 | 0.25 | 0.25 |
| Sodium citrate | 0.51 | 0.51 | 0.51 | 0.51 | 0.51 | 0.51 | 0.51 | 0.51 |
| Potassium sorbate | 0.18 | 0.18 | 0.18 | 0.18 | 0.18 | 0.18 | 0.18 | 0.18 |
| Methyl p-hydroxybenzoate | 0.13 | 0.13 | 0.13 | 0.13 | 0.13 | 0.13 | 0.13 | 0.13 |
| Xantural 75 | 0.4 | 0.4 | 0.4 | 0.4 | 0.4 | 0.4 | 0.4 | 0.4 |
| Glycerol | - | 10 | 10 | 10 | 7 | 14 | 14 | 7 |
| Sorbitol | 10 | - | 10 | 10 | 7 | 14 | 7 | 14 |
| Xylitol | 5 | 5 | - | 5 | 2 | 7 | 7 | 3 |
| Sucralose | 0.1 | 0.1 | 0.1 | - | 0.05 | 0.2 | 0.075 | 0.2 |
| Strawberry flavour | 0.12 | 0.12 | 0.12 | 0.12 | 0.12 | 0.12 | 0.12 | 0.12 |
| Mandarin flavour | 0.24 | 0.24 | 0.24 | 0.24 | 0.24 | 0.24 | 0.24 | 0.24 |
| Demineralized water | q.s. 100 | | | | | | | |

Formulation 8 was immediately found to be unsuitable because storage at 4°C led to formation of a crystallizate, on account of the excessive amount of the components of the sweetening system. Formulations 3-7 and 9-10 were submitted to the same palatability test described in Example 2, limited to the test for bitterness (the variations of the components of the sweetening system have no effect on the tests for tingling, clogging and viscidity). The values obtained with formulations 1 and 2 are also shown for comparison. The mean values obtained are presented in the following Table 7.

**TABLE 7**

| Composition | 1 | 2 | 3 | 4 | 5 | 6 | 7 | 9 | 10 |
|---|---|---|---|---|---|---|---|---|---|
| Bitterness | 3.5 | 3.3 | 9.3 | 9,2 | 9.1 | 9.8 | 4.4 | 3.5 | 3.8 |

Formulations 3 to 6 all proved to be very negative in the bitterness test, owing to absence of one of the four components of the sweetening system (sucralose, glycerol, sorbitol or xylitol). Formulation 7 was found to be only just acceptable, owing to the minimum content of all of the components of the sweetening system. Compositions 9 and 10 were found to be comparable with the values obtained for formulations 1 and 2.

The data in Tables 6 and 7 confirmed that only the sugar-free pharmaceutical formulations of the present invention, with simultaneous presence of the four components of the sweetening system in the appropriate amounts, have good palatability, comparable with the sucrose-based formulations.

## Claims

1. Sugar-free liquid pharmaceutical formulation comprising an aqueous solution of paracetamol, a solubilizing agent containing polyethylene glycol, a thickening agent containing xanthan gum, and a sweetening system containing sucralose and a mixture of polyols containing glycerol, sorbitol and xylitol in a total amount between 15% and 35% w/v relative to the total volume of said pharmaceutical formulation.

2. Pharmaceutical formulation according to Claim 1, **characterized in that** said solubilizing agent is a polyethylene glycol with a molecular weight greater than 1,000, present in an amount greater than 10% w/v relative to the total volume of said pharmaceutical formulation.

3. Pharmaceutical formulation according to Claim 2, **characterized in that** said solubilizing agent is a polyethylene glycol with a molecular weight between 3,000 and 8,000, present in an amount between 15% and 20% w/v relative to the total volume of said pharmaceutical formulation.

4. Pharmaceutical formulation according to any one of the preceding claims, **characterized in that** said thickening agent is a xanthan gum present in an amount between 0.1% and 2.0% w/v relative to the total volume of said pharmaceutical formulation.

5. Pharmaceutical formulation according to Claim 4, **characterized in that** said thickening agent is XANTURAL^{®}75 xanthan gum.

6. Pharmaceutical formulation according to any one of the preceding claims, **characterized in that** said sweetening system includes an amount of sucralose between 0.05% and 0.5% w/v relative to the total volume of said pharmaceutical formulation.

7. Pharmaceutical formulation according to any one of the preceding claims, **characterized in that** said sweetening system includes said mixture of polyols in an amount between 20% and 30% w/v relative to the total volume of said pharmaceutical formulation.

8. Pharmaceutical formulation according to Claim 7, **characterized in that** said sweetening system includes said mixture of polyols in an amount between 22% and 28% w/v relative to the total volume of said pharmaceutical formulation.

9. Pharmaceutical formulation according to any one of the preceding claims, **characterized in that** said sweetening system comprises from 7% to 14% w/v of glycerol, from 7% to 14% w/v of sorbitol, and from 3% to 7% w/v of xylitol relative to the total volume of said pharmaceutical formulation.

10. Pharmaceutical formulation according to any one of the preceding claims, **characterized in that** said pharmaceutical formulation comprises at least one further pharmaceutically acceptable additive selected from the group comprising preservatives, antioxidants, buffering agents, stabilizers, colorants and flavourings.

11. Pharmaceutical formulation according to Claim 10, **characterized in that** said pharmaceutically acceptable additive is present in an amount between 0.01% and 2.0% w/v relative to the total volume of said pharmaceutical formulation.

12. Pharmaceutical formulation according to any one of the preceding claims, **characterized in that** said pharmaceutical formulation comprises the ingredients of the following table in amounts between the minimum value and the maximum value expressed as a percentage w/v relative to the total volume of said pharmaceutical formulation:
| Ingredient | minimum | maximum |
|---|---|---|
| Paracetamol | 2.0 | 4.0 |
| PEG 6000 | 15.0 | 20.0 |
| Xanthan gum | 0.3 | 0.5 |
| Glycerol | 8.0 | 12.0 |
| Sorbitol | 8.0 | 12.0 |
| Xylitol | 4.0 | 6.0 |
| Sucralose | 0.08 | 0.12 |
| Buffering system | 0.50 | 1.00 |
| Preserving system | 0.25 | 0.35 |
| Strawberry flavour | 0.25 | 0.50 |
| Demineralized water | q.s. 100 | |

13. Pharmaceutically acceptable sugar-free liquid excipient for the administration of active principles of unpleasant taste comprising an aqueous vehicle, a solubilizing agent containing polyethylene glycol, a thickening agent containing xanthan gum, and a sweetening system containing sucralose and a mixture of polyols containing glycerol, sorbitol and xylitol in a total amount between15% and 35% w/v relative to the total volume of said pharmaceutical formulation.

14. Liquid excipient according to Claim 13, **characterized in that** said solubilizing agent has the characteristics according to either of Claims 2 and 3.

15. Liquid excipient according to either of Claims 13 and 14, **characterized in that** said thickening agent has the characteristics according to either of Claims 4 and 5.

16. Liquid excipient according to any one of Claims 13 to 15, **characterized in that** said sweetening system has the characteristics according to any one of Claims 6 to 9.

17. Liquid excipient according to any one of Claims 13 to 16, **characterized in that** said liquid excipient comprises at least one further pharmaceutically acceptable additive selected from the group comprising preservatives, antioxidants, buffering agents, stabilizers, colorants and flavourings.

18. Liquid excipient according to any one of Claims 13 to 17, **characterized in that** said liquid excipient comprises the ingredients of the following table in amounts between the minimum value and the maximum value expressed as a percentage w/v relative to the total volume of said liquid excipient:
| Ingredient | minimum | maximum |
|---|---|---|
| PEG 6000 | 15.0 | 20.0 |
| Xanthan gum | 0.3 | 0.5 |
| Glycerol | 8.0 | 12.0 |
| Sorbitol | 8.0 | 12.0 |
| Xylitol | 4.0 | 6.0 |
| Sucralose | 0.08 | 0.12 |
| Buffering system | 0.50 | 1.00 |
| Preserving system | 0.25 | 0.35 |
| Strawberry flavour | 0.25 | 0.50 |
| Demineralized water | q.s. 100 | |

## Patentansprüche

1. Zuckerfreie, wässrige pharmazeutische Formulierung, umfassend eine wässrige Paracetamollösung, ein Polyethylenglycol enthaltendes Solubilisierungsmittel, einen Xanthan-Gummi enthaltenden Verdicker und ein Süßungssystem, das Sucralose und ein Gemisch von Polyolen enthält, welches Glycerin, Sorbitol und Xylit in einer Gesamtmenge zwischen 15% und 35% m/V, bezogen auf das Gesamtvolumen der pharmazeutischen Formulierung, enthält.

2. Pharmazeutische Formulierung nach Anspruch 1, **dadurch gekennzeichnet, dass** das Solubilisierungsmittel ein Polyethylenglycol mit einem Molekulargewicht von mehr als 1.000 ist und in einer Menge von mehr als 10% m/V, bezogen auf das Gesamtvolumen der pharmazeutischen Formulierung, vorliegt.

3. Pharmazeutische Formulierung nach Anspruch 2, **dadurch gekennzeichnet, dass** das Solubilisierungsmittel ein Polyethylenglycol mit einem Molekulargewicht von 3.000 bis 8.000 ist und in einer Menge zwischen 15% und 20% m/V, bezogen auf das Gesamtvolumen der pharmazeutischen Formulierung, vorliegt.

4. Pharmazeutische Formulierung nach einem der vorhergehenden Ansprüche, **dadurch gekennzeichnet, dass** der Verdicker ein Xanthan-Gummi ist und in einer Menge zwischen 0,1% und 2,0% m/V, bezogen auf das Gesamtvolumen der pharmazeutischen Formulierung, vorliegt.

5. Pharmazeutische Formulierung nach Anspruch 4, **dadurch gekennzeichnet, dass** der Verdicker XANTURAL®75 Xanthan-Gummi ist.

6. Pharmazeutische Formulierung nach einem der vorhergehenden Ansprüche, **dadurch gekennzeichnet, dass** das Süßungssystem eine Menge an Sucralose zwischen 0,05% und 0,5% m/V, bezogen auf das Gesamtvolumen der pharmazeutischen Formulierung, enthält.

7. Pharmazeutische Formulierung nach einem der vorhergehenden Ansprüche, **dadurch gekennzeichnet, dass** das Süßungssystem das Gemisch von Polyolen in einer Menge zwischen 20% und 30% m/V, bezogen auf das Gesamtvolumen der pharmazeutischen Formulierung, enthält.

8. Pharmazeutische Formulierung nach Anspruch 7, **dadurch gekennzeichnet, dass** das Süßungssystem das Gemisch von Polyolen in einer Menge zwischen 22% und 28% m/V, bezogen auf das Gesamtvolumen der pharmazeutischen Formulierung, enthält.

9. Pharmazeutische Formulierung nach einem der vorhergehenden Ansprüche, **dadurch gekennzeichnet, dass** das Süßungssystem 7% bis 14% m/V Glycerin, 7% bis 14% m/V Sorbitol und 3% bis 7% m/V Xylit, bezogen auf das Gesamtvolumen der pharmazeutischen Formulierung, umfasst.

10. Pharmazeutische Formulierung nach einem der vorhergehenden Ansprüche, **dadurch gekennzeichnet, dass** die pharmazeutische Formulierung mindestens ein weiteres pharmazeutisch annehmbares Additiv, ausgewählt unter Konservierungsmitteln, Antioxidantien, Puffersubstanzen, Stabilisatoren, Färbemitteln und Aromastoffen, umfasst.

11. Pharmazeutische Formulierung nach Anspruch 10, **dadurch gekennzeichnet, dass** das pharmazeutisch annehmbare Additiv in einer Menge zwischen 0,01% und 2,0% m/V, bezogen auf das Gesamtvolumen der pharmazeutischen Formulierung, vorliegt.

12. Pharmazeutische Formulierung nach einem der vorhergehenden Ansprüche, **dadurch gekennzeichnet, dass** die pharmazeutische Formulierung die Inhaltsstoffe der folgenden Tabelle in Mengen zwischen dem Minimum-Wert und dem Maximum-Wert, ausgedrückt als % m/V bezogen auf das Gesamtvolumen der pharmazeutischen Formulierung, umfasst:
| Inhaltsstoff | Minimum | Maximum |
|---|---|---|
| Paracetamol | 2,0 | 4,0 |
| PEG 6000 | 15,0 | 20,0 |
| Xanthan-Gummi | 0,3 | 0,5 |
| Glycerin | 8,0 | 12,0 |
| Sorbitol | 8,0 | 12,0 |
| Xylit | 4,0 | 6,0 |
| Sucralose | 0,08 | 0,12 |
| Puffersystem | 0,50 | 1,00 |
| Konservierungssystem | 0,25 | 0,35 |
| Erdbeergeschmack | 0,25 | 0,50 |
| Vollentsalztes Wasser | q.s: 100 | |

13. Pharmazeutisch annehmbarer, zuckerfreier, flüssiger Exzipient zur Verabreichung von Wirkstoffen mit unangenehmem Geschmack, umfassend ein wässriges Vehikel, ein Polyethylenglycol enthaltendes Solubilisierungsmittel, einen Xanthan-Gummi enthaltenden Verdicker und ein Süßungssystem, das Sucralose und ein Gemisch von Polyolen enthält, welches Glycerin, Sorbitol und Xylit in einer Gesamtmenge zwischen 15% und 35% m/V, bezogen auf das Gesamtvolumen der pharmazeutischen Formulierung, enthält.

14. Flüssiger Exzipient nach Anspruch 13, **dadurch gekennzeichnet, dass** das Solubilisierungsmittel die Eigenschaften nach einem der Ansprüche 2 oder 3 aufweist.

15. Flüssiger Exzipient nach einem der Ansprüche 13 oder 14, **dadurch gekennzeichnet, dass** der Verdicker die Eigenschaften nach einem der Ansprüche 4 oder 5 aufweist.

16. Flüssiger Exzipient nach einem der Ansprüche 13 bis 15, **dadurch gekennzeichnet, dass** das Süßungssystem die Eigenschaften nach einem der Ansprüche 6 bis 9 aufweist.

17. Flüssiger Exzipient nach einem der Ansprüche 13 bis 16, **dadurch gekennzeichnet, dass** der flüssige Hilfsstoff mindestens ein weiteres pharmazeutisch annehmbares Additiv, ausgewählt unter Konservierungsstoffen, Antioxidantien, Puffersubstanzen, Stabilisatoren, Färbemitteln und Aromastoffen, umfasst.

18. Flüssiger Exzipient nach einem der Ansprüche 13 bis 17, **dadurch gekennzeichnet, dass** der flüssige Hilfsstoff die Inhaltsstoffe der folgenden Tabelle in Mengen zwischen dem Minimum-Wert und dem Maximum-Wert, ausgedrückt als m/V-%, bezogen auf das Gesamtvolumen des flüssigen Exzipienten, umfasst:
| Inhaltsstoff | Minimum | Maximum |
|---|---|---|
| PEG 6000 | 15,0 | 20,0 |
| Xanthan-Gummi | 0,3 | 0,5 |
| Glycerin | 8,0 | 12,0 |
| Sorbitol | 8,0 | 12,0 |
| Xylit | 4,0 | 6,0 |
| Sucralose | 0,08 | 0,12 |
| Puffersystem | 0,50 | 1,00 |
| Konservierungssystem | 0,25 | 0,35 |
| Erdbeergeschmack | 0,25 | 0,50 |
| Vollentsalztes Wasser | q.s. 100 | |

## Revendications

1. Formulation pharmaceutique liquide sans sucre comprenant une solution aqueuse de paracétamol, un agent solubilisant contenant du polyéthylèneglycol, un agent épaississant contenant de la gomme xanthane, et un système édulcorant contenant du sucralose et un mélange de polyols contenant du glycérol, du sorbitol et du xylitol en une quantité totale comprise entre 15 % et 35 % p/v par rapport au volume total de ladite formulation pharmaceutique.

2. Formulation pharmaceutique selon la revendication 1, **caractérisée en ce que** ledit agent solubilisant est un polyéthylèneglycol ayant une masse moléculaire supérieure à 1000, présent en une quantité supérieure à 10 % p/v par rapport au volume total de ladite formulation pharmaceutique.

3. Formulation pharmaceutique selon la revendication 2, **caractérisée en ce que** ledit agent solubilisant est un polyéthylèneglycol ayant une masse moléculaire comprise entre 3000 et 8000, présent en une quantité comprise entre 15 % et 20 % p/v par rapport au volume total de ladite formulation pharmaceutique.

4. Formulation pharmaceutique selon l'une quelconque des revendications précédentes, **caractérisée en ce que** ledit agent épaississant est une gomme xanthane présente en une quantité comprise entre 0,1 % et 2,0 % p/v par rapport au volume total de ladite formulation pharmaceutique.

5. Formulation pharmaceutique selon la revendication 4, **caractérisée en ce que** ledit agent épaississant est la gomme xanthane XANTURAL®75.

6. Formulation pharmaceutique selon l'une quelconque des revendications précédentes, **caractérisée en ce que** ledit système édulcorant contient une quantité de sucralose comprise entre 0,05 % et 0,5 % p/v par rapport au volume total de ladite formulation pharmaceutique.

7. Formulation pharmaceutique selon l'une quelconque des revendications précédentes, **caractérisée en ce que** ledit système édulcorant contient ledit mélange de polyols en une quantité comprise entre 20 % et 30 % p/v par rapport au volume total de ladite formulation pharmaceutique.

8. Formulation pharmaceutique selon la revendication 7, **caractérisée en ce que** ledit système édulcorant contient ledit mélange de polyols en une quantité comprise entre 22 % et 28 % p/v par rapport au volume total de ladite formulation pharmaceutique.

9. Formulation pharmaceutique selon l'une quelconque des revendications précédentes, **caractérisée en ce que** ledit système édulcorant comprend de 7 % à 14 % p/v de glycérol, de 7 % à 14 % p/v de sorbitol, et de 3 % à 7 % p/v de xylitol par rapport au volume total de ladite formulation pharmaceutique.

10. Formulation pharmaceutique selon l'une quelconque des revendications précédentes, **caractérisée en ce qu'**elle comprend au moins un autre additif pharmaceutiquement acceptable choisi dans le groupe comprenant les conservateurs, les antioxydants, les agents tampons, les stabilisants, les colorants, et les aromatisants.

11. Formulation pharmaceutique selon la revendication 10, **caractérisée en ce que** ledit additif pharmaceutiquement acceptable est présent en une quantité comprise entre 0,01 % et 2,0 % p/v par rapport au volume total de ladite formulation pharmaceutique.

12. Formulation pharmaceutique selon l'une quelconque des revendications précédentes, **caractérisée en ce qu'**elle comprend les ingrédients du tableau suivant en des quantités comprises entre la valeur minimale et la valeur maximale exprimées en pourcentage p/v par rapport au volume total de ladite formulation pharmaceutique :
| Ingrédient | minimum | maximum |
|---|---|---|
| Paracétamol | 2,0 | 4,0 |
| PEG 6000 | 15,0 | 20,0 |
| Gomme xanthane | 0,3 | 0,5 |
| Glycérol | 8, 0 | 12,0 |
| Sorbitol | 8,0 | 12,0 |
| Xylitol | 4,0 | 6, 0 |
| Sucralose | 0,08 | 0,12 |
| Système tampon | 0,50 | 1,00 |
| Système conservateur | 0,25 | 0,35 |
| Saveur fraise | 0,25 | 0,50 |
| Eau déminéralisée | q.s. 100 | |

13. Excipient liquide sans sucre pharmaceutiquement acceptable pour l'administration de principes actifs ayant un goût déplaisant, comprenant un véhicule aqueux, un agent solubilisant contenant du polyéthylèneglycol, un agent épaississant contenant de la gomme xanthane, et un système édulcorant contenant du sucralose et un mélange de polyols contenant du glycérol, du sorbitol et du xylitol en une quantité totale comprise entre 15 % et 35 % p/v par rapport au volume total de ladite formulation pharmaceutique.

14. Excipient liquide selon la revendication 13, **caractérisé en ce que** ledit agent solubilisant a les caractéristiques de l'une ou l'autre des revendications 2 et 3.

15. Excipient liquide selon l'une ou l'autre des revendications 13 et 14, **caractérisé en ce que** ledit agent épaississant a les caractéristiques de l'une ou l'autre des revendications 4 et 5.

16. Excipient liquide selon l'une quelconque des revendications 13 à 15, **caractérisé en ce que** ledit système édulcorant a les caractéristiques de l'une quelconque des revendications 6 à 9.

17. Excipient liquide selon l'une quelconque des revendications 13 à 16, **caractérisé en ce qu'**il comprend au moins un autre additif pharmaceutiquement acceptable choisi dans le groupe comprenant les conservateurs, les antioxydants, les agents tampons, les stabilisants, les colorants, et les aromatisants.

18. Excipient liquide selon l'une quelconque des revendications 13 à 17, **caractérisé en ce qu'**il comprend les ingrédients du tableau suivant en des quantités comprises entre la valeur minimale et la valeur maximale exprimées en pourcentage p/v par rapport au volume total dudit excipient liquide :
| Ingrédient | minimum | maximum |
|---|---|---|
| PEG 6000 | 15,0 | 20,0 |
| Gomme xanthane | 0,3 | 0,5 |
| Glycérol | 8,0 | 12,0 |
| Sorbitol | 8, 0 | 12,0 |
| Xylitol | 4,0 | 6, 0 |
| | | |
|---|---|---|
| Sucralose | 0,08 | 0,12 |
| Système tampon | 0,50 | 1,00 |
| Système conservateur | 0,25 | 0,35 |
| Saveur fraise | 0,25 | 0,50 |
| Eau déminéralisée | q.s. 100 | |
